# EUROPEAN PATENT APPLICATION

(11) **EP 2 052 733 A1**
(43) Date of publication of application: **29.04.2009**
(21) Application number: 07020966.3
(22) Date of filing: 26.10.2007
(51) Int. Cl.: A61K 36/48, A61P 17/02

(54) **Herbal extracts for treatment of chronic wounds**

(71) Applicant: Parsroos Co., Shahrak-e-Gharb, Tehran 14678-76744 (IR)
(72) Inventor: Farzamfar, Bardia, Shahrak-e-Ekbatan Tehran (IR); Madani, Hessameddin, Tehran (IR); Gharibdoust, Farhad, Tehran (IR); Farhadi, Mohammad Unit 2, No. 19, 2nd Alley,, Tehran (IR); Novitsky, Yuri Alexevich, Ryazan Province (RU); Khorramkhorshid, Hamid Reza, Sattarkhan Ave, Tehran (IR); Sadeghi, Behnam, Tehran (IR); Larijani, Bagher, Shariati Ave., Gholhak Tehran (IR)
(74) Representative: Winkler, Andreas Fritz Ernst

(57) **Abstract**

The present invention refers to a method for preparing a herbal extract from *Mellilotus sp. (Mellilotus officinalis)*, preferably comprising a treatment by pulsed electromagnetic field of high frequency. The herbal extract, optionally comprising selenium and/or urea and/or fructose and/or phosphoglycerol (or its sodium salt), is useful in the treatment of chronic wounds, in particular associated with states in which the normal wound repair ability is weakened, and preferably diabetic foot ulcers and/or bed sores.

## Description

The present invention refers to a method for preparation of a herbal extract from *Mellilotus officinalis,* optionally comprising a treatment by a pulsed electromagnetic field. The present invention further refers to a herbal extract prepared by said method, optionally comprising urea, fructose, selenium and/or phosphoglycerol or its sodium salt, and to its use for the treatment of chronic wounds, preferably diabetic foot ulcers and/or bed sores. The present invention also refers to a pharmaceutical composition and to a kit.

### Background of the invention

Diabetes is one of the main problems of human being for centuries. This disease is represented by loss of insulin secretion from pancreatic beta cells and/or impairment of insulin receptors on the surface of the peripheral cells; both will show paraclinically high concentration of glucose in blood stream.

Only in the United States, an estimated 18.2 million people, 6.3 percent of the population, have diabetes, a serious, lifelong condition. Of those, 13 million have been diagnosed. About 5.2 million people have not yet been diagnosed. Each year, about 1.3 million people of age 20 or older are diagnosed with diabetes.

Diabetes is widely recognised as one of the leading causes of death and disability in the world. In 2000, it was the sixth leading cause of death in USA. However, diabetes is likely to be underreported as the underlying cause of death on death certificates. About 65 percent of deaths among those with diabetes are attributed to heart disease and stroke.

Diabetes is associated with long-term complications that affect almost every part of the body. The disease often leads to blindness, heart and blood vessel diseases, strokes, kidney failure, amputations, and nerve damage. Uncontrolled diabetes can complicate pregnancy, and birth defects are more common in babies born to women with diabetes.

In 2002, diabetes costs in the United States totaled $132 billion. Indirect costs, including disability payments, time lost from work, and premature death, totaled $40 billion; direct medical costs for diabetes care, including hospitalisations, medical care, and treatment supplies, totaled $92 billion.

Diabetes has short term as well as long term adverse effects. Control of blood sugar levels is the best preventive treatment to decrease and postpone long-term adverse effects of diabetes, but this is not always possible, and depending on several factors, including duration of high blood sugar level, physiology and race of patient and severity of the disease, long-term adverse effects will appear. Three major long-term adverse effects are retinopathy, nephropathy and neuropathy. Retinopathy will affect the retina and will cause loss of vision in the final stages of the disease. Nephropathy will affect the kidneys and cause their malfunctions and neuropathy will cause loss of sense in the limbs, mainly feet. As a secondary adverse effect, neuropathy of foot nerves will cause creation of wounds usually infected with bacteria and other opportunistic infections. Treatment of these wounds usually is not successful and finally will result in partial or total amputation of foot.

After amputation, the diabetic patient will be a disabled person, besides several other problems arising from diabetes. Disability has social, and economical side effects on human beings.

Among people with diabetes, 15% will experience a foot ulcer in their lifetime; foot ulcer is a major predictor of future lower-extremity amputation in patients with diabetes. Indeed, about 14-24% of people with a foot ulcer will require an amputation. It is therefore not surprising that diabetes is the leading cause of non-traumatic lower-extremity amputations. Despite many efforts in order to prevent amputation in the last decade, the incidence of lower-extremity amputation in people with diabetes is rising. Thus, appropriate techniques for wound care that can reduce amputation rates are essential for prevention strategy.

Healing of chronic wounds such as diabetic ulcers or bed sores is an important clinical problem. The known methods of accelerating chronic wound healing include:
- pressure relief (Armstrong DG, et al., "Off-loading the diabetic foot wound, a randomized clinical trial", Diabetes Care 2001, 24, 1019-1022; Ha Van G, et al., "Nonremovable, windowed, fibroglass cast boot in the treatment of diabetic plantar ulcers: Efficacy, safety, and compliance", Diabetes Care 2003, 26, 2848-2852; Mueller MJ, et al., "Effect of Achilles tendon lengthening on neuropathic plantar ulcers. A randomized trial", J. Bone Joint Surg. 2003, 85A, 1436-1445; Moffat CJ., et al., "Randomized trial of four-layer and two-layer bandage system in the management of chronic venous ulceration", Wound Rep. Reg. 2003, 11, 166-171),
- hydrotherapy,
- ultrasonic treatment,
- electrical stimulation (Peters EJ, et al., "Electric stimulation as an adjunct to heal diabetic foot ulcers: a randomized clinical trial", Arch. Phys. Med. Rehabil. 2001, 82, 721-725),
- hyperbaric oxygenation,
- vacuum assisted closure therapy,
- skin grafts and artificial skin replacements as well as methods from the tissue engineering field (Marston WA, et al., "The efficacy and safety of Dermagraft in improving the healing of chronic diabetic foot ulcers", Diabetes Care 2003, 26, 1701-1705),
- surgical venous rehabilitation (Bianchi C, et al., "Subfascial endoscopic perforator vein surgery combined with saphenous vein ablation: Results and critical analysis", J. Vasc. Surg. 2003, 38, 67-71),
- gene therapy (Yamasaki K, et al., "Reversal of Impaired Wound Repair in iNOS-deficient Mice by Topical Adenoviral-mediated iNOS Gene Transfer", J. Clin. Invest., Vol. 101, No. 5, March 1998, 967-971),
- use of protease regulating wound dressings (Cullen B, et al., "Mechanism of action of Promogran, a protease modulating matrix, for the treatment of diabetic foot ulcers", Wound Rep. Reg. 2002, 10, 16-25),
- use of growth factor-loaded gels, such as PDGF-BB (Regranex) or EGF (Tsang MW, et al., "Human Epidermal Growth Factor enhances healing of diabetic foot ulcers", Diabetes Care 2003, 26, 1856-1861),
- and finally local application of hydrogels that release NO (Bohl Masters KS, et al., "Effect of nitric oxide releasing poly(vinyl alcohol) hydrogel dressings on dermal wound healing in diabetic mice", Wound Rep. Reg. 2002, 10, 286-294).

The known methods for treatment of chronic wounds nevertheless have some disadvantages and drawbacks. Namely, some of them are very expensive and for that reason not commonly available, some of them demand regimens which are very difficult or painful for a patient, and quite frequently they are simply not sufficiently effective since chronic wounds are characterised by a very high recurrence rate.

Despite the great advances in the treatment of chronic wounds, there is still a need for a treatment which allows the chronic wounds to heal completely with a relatively low recurrence rate.

Consequently, there is a need for alternative, improved and superior pharmaceutical means providing accelerated healing of chronic wounds with decreased recurrence rate, which would allow avoiding any surgery intervention.

Therefore, it is an objective of the present invention to provide useful pharmaceutical means for the treatment of chronic wounds, preferably diabetic foot ulcers and/or bed sores, which would be effective (i.e. with a minimised risk of recurrence) and easy to administer.

Surprisingly, the herbal extract according to the present invention can cure this type of chronic wounds, such as diabetic foot ulcers, neuropathic ulcers, including neuropathic forefoot ulcers, diabetic pressure ulcers or diabetic venous ulcers.

Furthermore, the herbal extract according to the present .invention can be also used for treatment of bed sores which affect people who stay in one place for an extended period of time for any reason.

In general, the invention provides for a herbal extract which can be used for treatment of any chronic wounds associated with states such as diabetes, but also in patients in their older age or during steroid treatment, whilst the normal wound healing ability is seriously weakened.

Advantageously the herbal extract according to the present invention provides full healing with accelerated wound closure, and unexpectedly it also improves the quality of the tissue in the healing wound with very efficient hair growth on the scars. Obtaining healthier scar tissue ensures the lower rate of ulcer recurrence in the future.

In the prior art, melilot (*Mellilotus*), also known as sweet clover, is acknowledged as helpful in some disorders when administered internally or externally.

German Patent Application DE 10 2004 022 746 describes a dry composition of a few components in a tablet form,for oral administration comprising acetylsalicylic acid and dry extracts of sweet clover (i.e. melilot) leaves, horse chestnut seeds and ruscus roots for treatment of thrombosis.

European Patent EP 1 214 084 describes a multi-component anti-oxidant composition comprising a biflavone extract of Ginkgo biloba, leucocyanidine, Mellilotus and/or Aesculus extracts, Centella extract and Fucus extract for oral administration which is useful for treatment of circulation problems, such as phlebitis, varicose veins, arteriosclerosis, haemorrhoids and high blood pressure, as well as localised adiposity problems especially in men (so called "spare tyres") but also in women (cellulite), i.e. in the treatment of surplus fat.

European Patent EP 1 214 085 describes a multi-component anti-oxidant composition comprising a biflavone extract of Ginkgo biloba, leucocyanidine, Mellilotus and/or Aesculus extracts, and Centella extract for oral administration that is useful for treatment of circulation and chronic degenerative problems caused by damage to the vascular endothelium, the extracellular matrix or to surrounding tissues of the arterial, venous or lymphatic systems and hypertension.

European Patent Application EP 1 426 030 describes a cosmetic or dermatological compositions for external application comprising a Mellilotus extract along with many other plant extracts which are useful for preventing or ameliorating ageing of the skin.

Japanese Patent Application JP 2001322943 describes a multi-herbal therapeutic agent comprising amongst others and optionally, a Mellilotus extract that is useful for treatment of pimples.

US Patent Application US 2004/0156873 describes a multi-component topical composition comprising a Mellilotus extract as one of many blood micro-circulation improving agents for treatment of skin problems such as acne and rosacea.

International Publication WO 98/51291 describes a pharmaceutical composition comprising, amongst others, coumarine and/or sweet clover (i.e. melilot) and rutin extracts as an active compound acting on patient's mitochondrial membrane protein complexes, and useful for treatment of ischemia and/or pathalogies associated with ischemia or energy deficiency.

None of the prior art documents, however, proposes using a herbal extract of *Mellilotus sp.* for treatment of chronic wounds, and especially diabetic foot ulcers and/or bed sores.

### Summary of the invention

The object of the present invention is solved by a method for preparing a herbal extract, comprising the following steps:
(a) providing a plant material derived from *Mellilotus sp.*;
(b) drying the plant material;
(c) adding an organic solvent;
(d) incubating the mixture of plant material and organic solvent; and
(e) obtaining the herbal extract.

In one embodiment, the plant material is derived from *Mellilotus officinalis.*

Preferably, the plant material derived from *Mellilotus officinalis* comprises leaves and/or small stems.

In one embodiment, the drying in step (b) is carried out at a temperature in the range of about 20 to 50°C, preferably at about 37 to 45°C, most preferably at about 42°C.

Preferably, the drying in step (b) is carried out for a time period of about 3 to 4 days.

In one embodiment, the organic solvent is ethanol, preferably of about 60 to 96 % (v/v), more preferably of about 80 to 96 % (v/v), most preferably of about 96 % (v/v).

In one embodiment, the incubating in step (d) is carried out for a time period in the range of about 10 to 40 days, preferably of about 22 to 38 days, most preferably of about 25 to 35 days.

In one embodiment, the incubating in step (d) is carried out at a temperature in the range of about 20 to 50°C, preferably of about 37 to 45°C, most preferably of about 42°C.

In one embodiment, the method additionally comprises the following step:
(f) adding urea.
   In one embodiment, the method additionally comprises the following step:
(g) adding fructose.
   In one embodiment, the method additionally comprises the following step:
(h) adding phosphoglycerol and/or its sodium salt.
   In one embodiment, the method additionally comprises the following step:
(i) adding selenium and/or an organic or inorganic salt thereof.
   Preferably, the selenium or salt thereof is added to a concentration of free selenium in the range of about 1 to 100 mg/l, preferably of about 5 to 50 mg/l, most preferably of about 10 to 20 mg/l.

In one embodiment, the method additionally comprises the following step:
(j) exposing the herbal extract to a pulsed electromagnetic field.

In one embodiment, the electromagnetic field pulse has a sinusoidal, rectangular and/or stochastic shape.

In one embodiment, the pulsed electromagnetic field has a frequency in the range of about 5 to 750 kHz, preferably of about 50 to 350 MHz, most preferably of about 250 MHz.

In one embodiment, the pulsed electromagnetic field has a power in the range of about 10 to 200 Watt, preferably of about 20 to 100 Watt, most preferably of about 45 Watt.

In one embodiment, the pulsed electromagnetic field has a magnetic field strength in the range of 100 to 150 µTesla.

In one embodiment, the exposing in step (j) is carried out for a time period of about 3 to 5 minutes.

In one embodiment, the exposing in step (j) is repeated, and is preferably carried out for three times.

The object of the present invention is further solved by a herbal extract obtainable by the method according to the present invention.

The object of the present invention is further solved by a use of the herbal extract according to the present invention or prepared by the method according to the present invention, for the treatment of chronic wounds in a subject.

The object of the present invention is further solved by a use of the herbal extract according to the present invention or prepared by the method according to the present invention, for the manufacture of a pharmaceutical composition for the treatment of chronic wounds in a subject.

In one embodiment, the chronic wounds are associated with states in which the normal wound repair ability is weakened.

In one embodiment, the chronic wounds are associated with states such as diabetes and/or occur in patients in their older age or during steroid treatment.

In one embodiment, the chronic wounds are diabetic foot ulcers, neuropathic ulcers including neuropathic forefoot ulcers, diabetic pressure ulcers or diabetic venous ulcers; bed sores or pressure ulcers associated with long-term disability.

In one embodiment, the chronic wounds are diabetic foot ulcers.

In one embodiment, the chronic wounds are bed sores.

In one embodiment of the use, the subject is a vertebrate, preferably a mammal, most preferably a human.

In one embodiment, the subject is not pregnant.

The object of the present invention is further solved by a pharmaceutical composition, comprising the herbal extract according to the present invention or prepared by the method according to the present invention.

In one embodiment, the pharmaceutical composition additionally comprises a pharmaceutically acceptable carrier.

In one embodiment, the pharmaceutical composition is formulated for administration by injection.

In one embodiment, the pharmaceutical composition is formulated for oral administration.

In one embodiment, the pharmaceutical composition is formulated for topical administration.

The object of the present invention is further solved by a kit comprising the pharmaceutical composition according to the present invention.

The term "chronic wounds" as used herein refers to clinical conditions having characteristic symptoms wherein the wounds that do not heal for a prolonged time and frequently have a strong tendency to recurrence. The chronic wounds usually are associated with states in which the normal wound healing ability is weakened, such as diabetes, patients in their older age, patients with limited ability of movement, or patients during steroid treatment. Thus, patients with such badly healing wounds are those suffering from diabetic ulcers, including diabetic foot ulcers, diabetic neuropathic ulcers, including neuropathic forefoot ulcers, diabetic pressure ulcers or diabetic venous ulcers, as well as patients with limited movement ability suffering from bed sores. Bed sores usually affect peoples who stay in one place for prolonged time for any reason.

The term "stochastic shape" herein has the meaning that the electromagnetic field pulse is in the form of a noise. Preferably, the electromagnetic field pulse is of rectangular shape and is combined with a sinusoidal wave inside. The "power" (Watt) of the pulsed electromagnetic filed means e.g. the effective power. The value of the "magnetic field strength" (Tesla) of the pulsed electromagnetic field is indicated e.g. from peak to peak.

The term "pharmaceutical composition", as used herein, is intended to comprise the herbal extract of the present invention. A pharmaceutical composition comprising at least one pharmaceutically active component of the herbal extract of the present invention and/or at least one derivative or analogue of said active component and corresponding salts thereof is also considered.

The pharmaceutical composition can be, for example, in a liquid form, e.g. a solution, syrup, elixir, emulsion and suspension, or in a solid form, e.g. a capsule, caplet, tablet, pill, powder, and suppository. Granules or semi-solid forms and gelcaps are also considered. In case that the pharmaceutical composition is a liquid or a powder, the dosage unit optionally is to be measured, e.g. by a teaspoonful. In addition to the herbal extract or the pharmaceutically active component, the pharmaceutical composition can comprise, for example, flavouring agents, sweeteners, dyes, preservatives, stabilizers, colouring agents, diluents, suspending agents, granulating agents, lubricants, binders and disintegrating agents. A tablet, for example, can be coated. Injectable liquids should be sterile. Transdermal delivery systems and liposomal systems are also considered. All of the formulations mentioned can be intended for immediate release, timed release and sustained release.

The term "pharmaceutically acceptable", as used herein, means at least non-toxic. The "pharmaceutically acceptable carrier", as meant in the present disclosure, may take a wide variety of forms depending upon the desired route of administration. The term comprises conventional pharmaceutical diluents such as water or ethanol and conventional tabletting or encapsulation ingredients such as corn starch, lactose, sucrose, sorbitol, talc, stearic acid, magnesium stearate, dicalcium phosphate, gelatine capsules or gums.

The pharmaceutical composition of the present invention can be administered via different routes, such as oral, sublingual, parenteral, intravenous, intraperitoneal, nasal, vaginal, rectal, subcutaneous, intradermal, intramuscular and topical. A dosage unit can be administered once or several times a day, week or month. The delivery can also be continuously, for example by infusion or through a transdermal sustained release system.

Thus, the present invention provides an herbal extract from *Mellilotus officinalis* optionally treated by electromagnetic field radiation. Clinical data showed a beneficial effect of the extract in the treatment of chronic wounds, such as diabetic foot ulcers and/or bed sores. Studies conducted both *in vitro* and in experimental animal models revealed that the extract does not exert toxicity, mutagenicity, or oncogenicity. Pregnancy, however, is a contraindication.

### Brief description of drawings

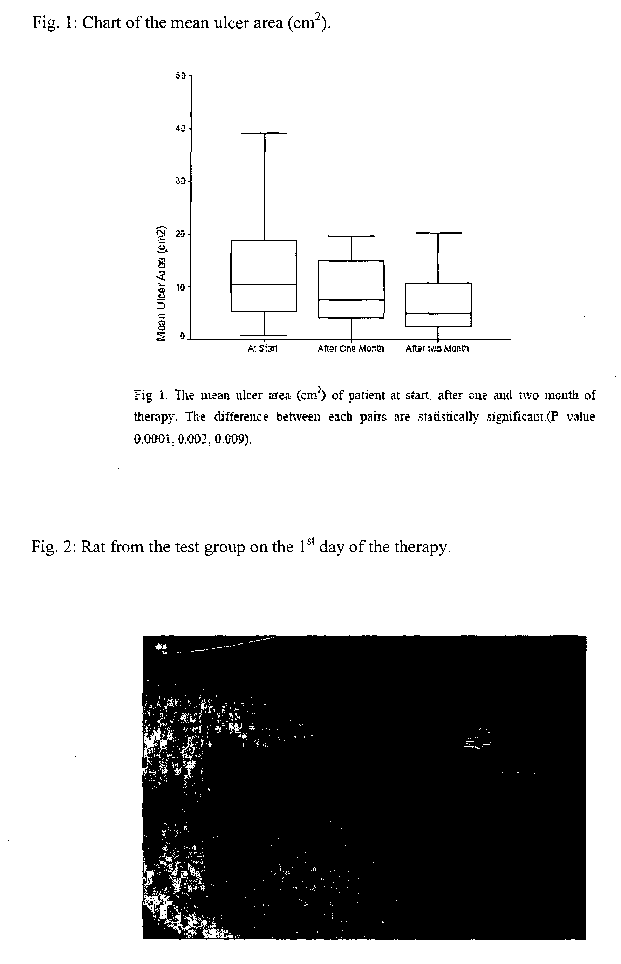

Fig. 1 presents a chart of the mean ulcer area (cm²) of a patient at start, after one and two months of the therapy. The differences between each pair are statistically significant (P value 0.0001, 0.002, and 0.009). The herbal extract was administered by injection and as a topical ointment to human patients.
Figures 2 to 5 show photos which were taken during in vivo testing of the herbal extract according to the present invention on dermal wounds in the diabetic rat model. The herbal extract was applied in a form of dressings containing a solution thereof covering the wounds of the test group animals.
Fig. 2 presents a picture of a rat from the test group taken on the 1^{st} day of therapy.
Fig. 3 presents a picture of a rat from the test group taken on the 22^{nd} day of therapy. The wound is completely healed and the new, long fur covers the entire scar area. No signs of the previous wound could be seen.
Fig. 4 presents a picture of a rat from the control group taken on the 1^{st} day of testing.
Fig. 5 presents a picture of a rat from the control group taken on the 22^{nd} day of testing. The wound is closed but the scar is still remarkably severe and completely naked.
Figures 6 to 13 show photos which have been taken during clinical trials of the herbal extract according to the present invention on diabetic foot ulcers of human subjects.

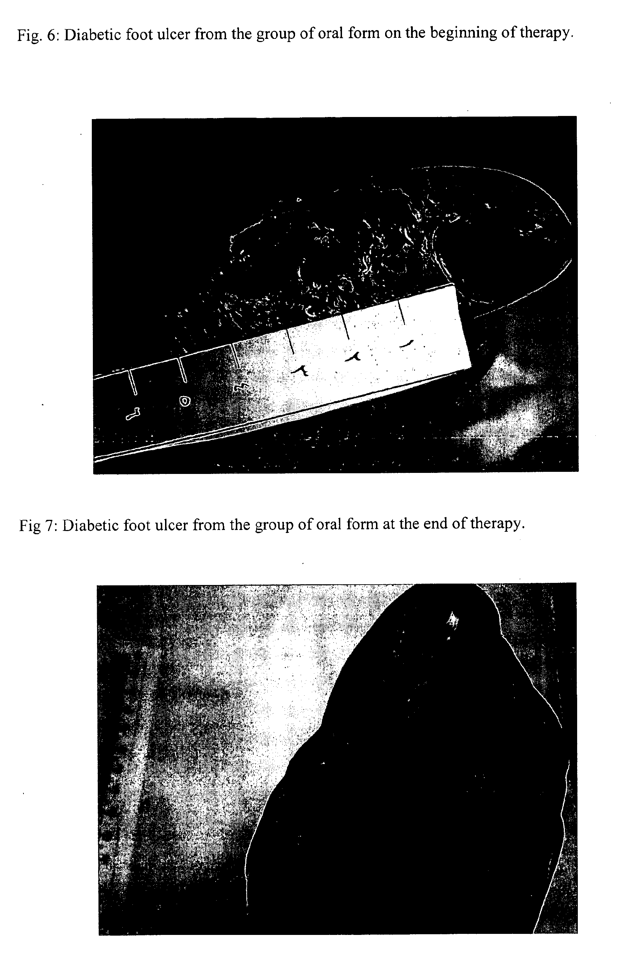

Fig. 6 presents a picture of a diabetic foot ulcer from the group of oral form of herbal extract taken on the beginning of therapy.
Fig. 7 presents a picture of the diabetic foot ulcer from the above patient in the group of oral form of herbal extract taken at the end of therapy.

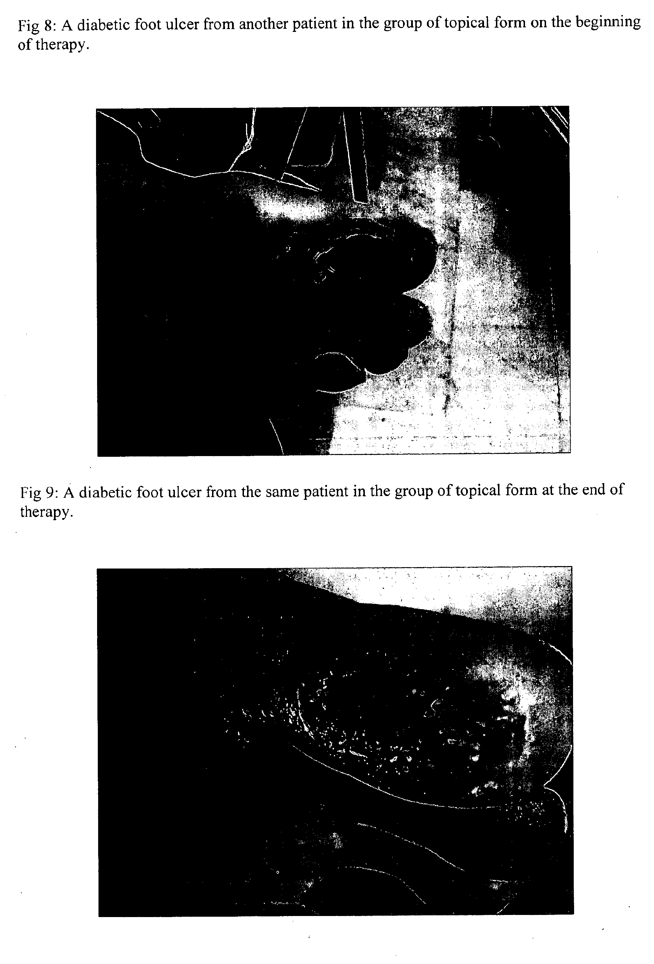

Fig. 8 presents a picture of another diabetic foot ulcer from the group of topical form of herbal extract taken on the beginning of therapy.
Fig. 9 presents a picture of the diabetic foot ulcer from the above patient in the group of topical form of herbal extract taken at the end of therapy.

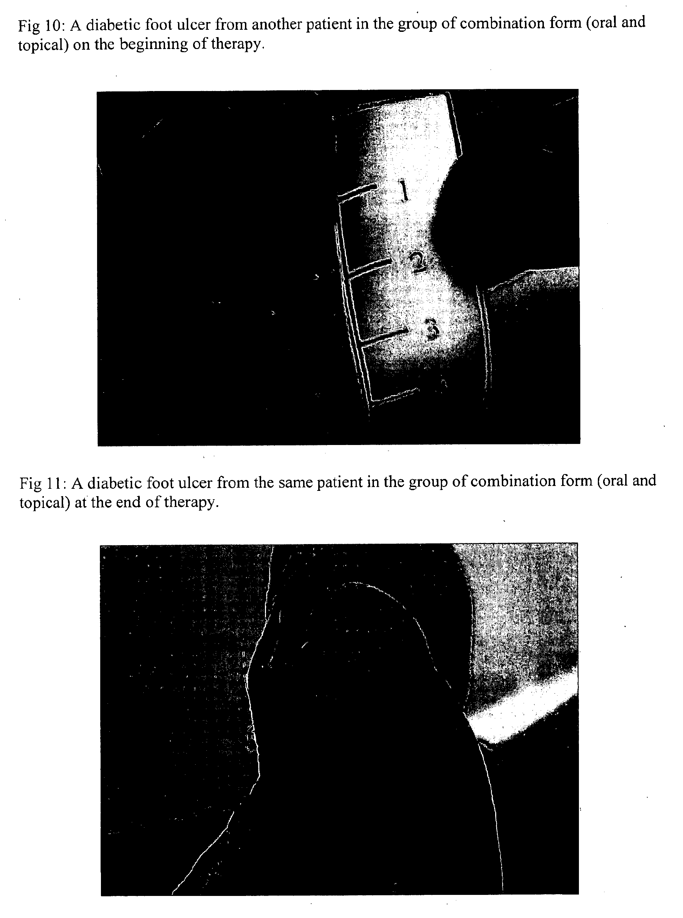

Fig. 10 presents a picture of a diabetic foot ulcer from the group of combination form of oral and topical herbal extract taken on the beginning of therapy.
Fig. 11 presents a picture of the diabetic foot ulcer from the above patient in the group of combination form of oral and topical herbal extract taken at the end of therapy.

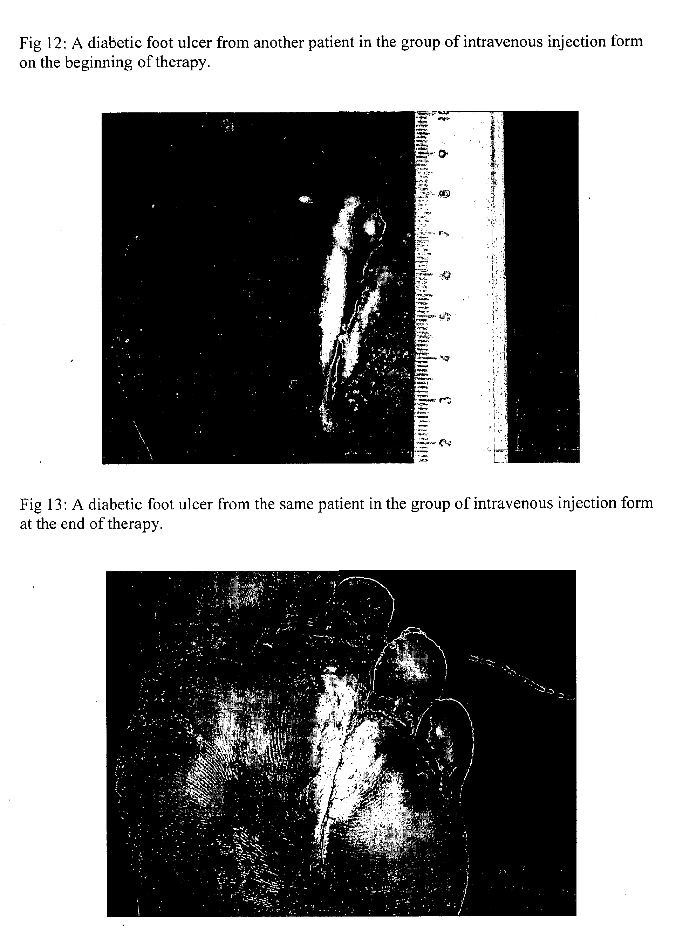

Fig. 12 presents a picture of another diabetic foot ulcer from the group of intravenous injection form of herbal extract taken on the beginning of therapy.
Fig. 13 presents a picture of the diabetic foot ulcer from the above patient in the group of intravenous injection form of herbal extract taken at the end of therapy.

### Detailed description of the invention

The invention will now be described in more detail by the following examples with the intention to exemplify the invention. The examples, however, are not intended to have any limiting effect on the subject-matter of the claims or on the scope of protection.

### EXAMPLE 1: Preparation of raw herbal extracts

Leaves and small stems of *Mellilotus officinalis* are collected from wild fields. After separation of useful parts and initial cleaning, the material is dried on a wooden network in a dark place for 3-4 days. In dried condition, the plant material should be green without any change in colour, and leaves and stems should be brittle. For extraction, airtight glass vessels are used. The dried plant material is broken into small pieces (2-5 cm) and placed into the glass vessels such that there is no space left. After packing (compressing), EtOH (96 % v/v) is added until the vessel is filled completely. The vessels are placed into an incubator (37 to 45°C, preferably 42°C) for 10-40 days until a dark green solution appears.

After 10-40 days, when the plant material is colourless, to each litre of the *Mellilotus officinalis* extract, respectively, 50 mg urea, 700 mg fructose, 16 mg selenium and 1000 mg of phosphoglycerol sodium salt are added. Then, the vessels are sealed again and kept in the incubator for 24 h at 42°C. The extracts are collected by separating them from the plant material using a cloth filter.

### EXAMPLE 2: Electromagnetic treatment

After incubation, the vessels with the extracts of Example 1 are exposed 4 times to an electromagnetic field, 3 min each, and are pooled together. The resulting extract is passed sequentially through a 5, 0.45 and 0.22 µm filter, respectively, and distributed to sterile vials. After labelling and packaging, the herbal extract is ready for use.

The electromagnetic field, to which the raw extracts are exposed, is pulsed, powerful and monopolar because the direction of the electric current generated in a Magnetic Impulse Generator (MIG) apparatus does not change. The pulsed magnetic field has a very high frequency ranging from 5 kHz to 750 kHz. In this example, the pulse of a rectangular shape is used. Nevertheless, a sinusoidal or stochastic shape is considered as well. Preferably, the pulse is of rectangular shape and is combined with a sinusoidal wave inside. It is hypothesized that the special kind of the produced pulse causes some changes in the physical configuration of atoms in the molecules and/or arrangements of molecules and thus leads to altered chemical properties.

The herbal extract is exposed to an electromagnetic field 3 times, 2 to 5 min each, preferably 5 min. The electrical power of the pulses used is about 20 to 100 Watt, and the best effect is obtained with 45 Watt.

### EXAMPLE 3: Pre-clinical studies - tests on toxicity

In pre-clinical studies, the herbal extract of the present invention according to Example 2 was examined for acute toxicity and chronic toxicity. For studying the drug acute toxicity, BALB/c mice and Wistar rats were injected with a single i.m. injection. The drug chronic toxicity was studied during 3 months in Wistar rats and 1 month in dogs. The potential mutagenic, embryotoxic, teratogenic, allergenic and immunotoxic properties of the herbal extract as well as its effect on the reproductive function were also investigated.

### Conclusions on toxicity:

Summing up the results of the toxicity studies on the herbal extract of Example 2, we can note that the preparation was non-toxic at a single i.m. injection of the herbal extract to laboratory animals and was well tolerated by Wistar rats and dogs during i.m. injection.

The investigation showed that a single i.m. injection of the herbal extract diluted 1:10 in normal saline in doses of 0.5-1.0 ml per mouse (BALB/c mice) does not cause intoxication and death of the animals. Increasing the administered dose to 1.5-2.0 ml per mouse (75-100 ml/kg) led to a decrease in motor activity and depression of animals, but no death was observed.

Intramuscular injection of the herbal extract dissolved 1:5 in normal saline to BALB/c mice was followed by pain, profound depression of animals and their death. Intramuscular and intraperitoneal injection of the herbal extract to BALB/c mice, in doses close to LD₅₀, was accompanied by depression, narcosis and sleep in animals. The intoxication pattern of the herbal extract in mice at LD₅₀, was similar to their poisoning by ethyl alcohol which is present in the herbal extract as vehicle. According to toxicity indices, the herbal extract was proved to be low toxic when administered intramuscularly and intraperitoneally at LD₅₀ levels (42-52 ml/kg) to BALB/c mice and Wistar rats. No specific and sex-related differences were observed in the sensitivity of the given animal species to the toxic effects of the preparation. In addition, there was no significant difference between intramuscular and intraperitoneal ways of administration in BALB/c mice.

Chronic toxicity experiment was carried out in Wistar rats daily for 3 months by intramuscular injection of 0.07 and 0.21 ml/kg of preparation and in dogs daily for 1 month by intramuscular injection of 0.07 ml/kg of the herbal extract. The mentioned doses of the preparation were diluted 1:10 in sterile normal saline before injection.

Results of study showed that doses of 0.07 and 0.21 ml/kg of the herbal extract in a 3 month chronic experiment in Wistar rats and 0.07 ml/kg for 1 month chronic experiment in dogs are well tolerated by animals and do not influence hematologic indices, functional state of the main organs of test animals (according to the data of the biochemical tests used and ECG). No pathomorphological changes of the internal organs or toxicoallergenic reaction were observed in these groups of animals after the end of chronic toxicity experiments. Long term i.m. injection of the preparation in doses of 0.07 and 0.21 ml/kg in Wistar rats and 0.07 ml/kg in dogs diluted 1:10 in normal saline did not show locally irritating effects.

According to requirements of Pharmacological State Committee of Ministry of Public Health an investigation of mutagenic properties of the herbal extract was conducted.

The ability of the preparation to induce gene mutations in indicator microorganism *Salmonella typhimurium* in Ames test, induce chromosomal aberrations in the bone marrow cells of hybrid F1(CBA*C₅₇B1₆) mice, cause dominant lethal mutation in embryonic mice cells and system of DNA repair in E. coli PQ 37 in SOS-chromotest was studied.

The results of these investigations revealed that the herbal extract does not have any mutagenic properties.

Intramuscular injection of the herbal extract in dose of 0.21 ml/kg to pregnant Wistar rats did not influence the increase of the body weight of pregnant females as compared to control group during all pregnancy period. Duration of pregnancy, number of live fetuses,

implantation sites, yellow bodies and embryo's body weight in experimental animals did not show significant difference compared to control group. The index of pre-implantational death in experimental group did not change significantly while post-implantational death was lower in the experimental group compared to the control group.

During a macroscopic examination and microscopic investigation of standard sections of foetuses, that underwent the influence of 0.21 ml/kg of the herbal extract during the prenatal period, in 6.7% of all the cases underdevelopment of the foetuses was observed. The effect can be evaluated as a defect of embryo's development.

Analysis of the prepared sections stained with alizarin that was necessary for studying the development of bone system in rat foetuses exposed to 0.21 ml/kg of the herbal extract during the prenatal period, did not show defects of skeleton development. But at the same time a delay of ossification in the majority of points of calcification was observed.

Under the influence of intramuscular injection of 0.21 ml/kg of the herbal extract from the 1^{st} to the 20^{th} days of gestation a decrease in the number of new born rats and increase in the number of stillborn rats was noticed as compared with the control group. The rats' body weight exposed to the herbal extract during the prenatal period was lower than the indices of the control group. Results of experiment on development of the posterity showed a retardation of physical development.

Therefore, the experiments conducted to establish that an intramuscular injection of 0.21 ml/kg of the herbal extract from the 1^{st} to the 20^{th} days of gestation has an embryotoxic and teratogenic effect in animals exposed to the herbal extract. Therefore, pregnancy can be considered as a contraindication for prescription of the herbal extract.

At a daily intramuscular injection of 0.21 ml/kg of the herbal extract to male rats during 10 weeks and female rats during 2 weeks the influence of the preparation on the reproductive function of animals was not established.

Studying the allergenic properties of the herbal extract on guinea-pigs showed that intramuscular injection of the herbal extract in doses of 0.07 and 0.14 ml/kg, administered 5 times with an interval of one day for sensitizing, and intraperitoneal injection of a determined dose of 0.14 ml/kg of the herbal extract at the 14^{th} and 21^{st} days after sensitization, the preparation does not cause anaphylactic shock.

The herbal extract in studied doses and schedules of sensitization does not have allergenic effect of delayed type hypersensivity reaction in guinea-pigs and in the reaction of the popliteal lymphnode in mice.

In doses of 0.07 ml/kg and 0.18 ml/kg herbal extract does not influence the number of antibody- forming and nucleus-containing cells in the spleen and it does not cause the delayed-type hypersensitivity reactions in mice. The data are evidence of the absence of negative influence of the herbal extract on the humoral and cellular immunity and therefore the absence of immunotoxicity of the preparation.

Based on all of the experiments conducted during the pre-clinical studies testing the toxicity of the herbal extract according to the present invention and the obtained results, the herbal extract is recommended for clinical trials with only contraindication in pregnancy.

### EXAMPLE 4: Effects of the herbal extract solution on dermal wound healing in diabetic rats

### Summary

Healing of chronic wounds such as diabetic ulcers is a significant clinical problem. Methods of accelerating healing include use of growth factor-loaded gels, hyperbaric oxygen, grafts, and artificial skin replacements. This study examines the *in vivo* response to a novel natural mixture comprising the herbal extract according to the present invention. The preliminary animal study on a diabetic rat model with impaired wound healing was conducted comparing the herbal extract according to the present invention with distilled water. As a result, the time to complete closure of wounds was lower in the herbal extract-treated group. The difference in wound healing since day 9^{th} of the treatment was apparent. The herbal extract-treated animals had lower scar tissues and the fur growth was complete while in water-treated animals a scar with impaired fur growth was apparent. The results of this study suggest that dermal use of this novel herbal extract has a potential to modulate wound healing and stimulate fur growth.

### Introduction

Chronic wounds such as pressure ulcers, diabetic foot ulcers, and venous ulcers make up approximately 70% of all skin wounds. Chronic wounds like these incur a huge cost and impair quality of life for millions of people. Approximately one in every five diabetic foot ulcers ultimately leads to amputation. A wound is considered chronic or non-healing if it does not heal in an orderly or timely sequence, or if the healing process does not result in structural integrity. Wounds that heal improperly may not possess the necessary mechanical integrity to remain healed.

Regarding the role of free radicals in the pathogenesis of diabetes and considering very high potential of the novel herbal extract in scavenging free radicals and its antioxidant power (Biomed Pharmacother. 2005 Aug; 59(7): 365-73. Review.), we aimed to examine how dermal exposure to this herbal extract may alleviate healing in a rat model of diabetic wounds.

### Methods

The animal model used for *in vivo* testing of the novel herbal extract (according to Example 2) was a full-thickness wound in the dorsal skin of diabetic rats. Wistar rats weighing 200-250 g from animal house of PSRC/TUMS were used. Animals were caged in separate cages. Diabetes was induced by administration of streptozotocin (Sigma-Aldrich, UK). Streptozotocin was administered at a dose of 55 mg/kg intraperitoneally. Before the administration of streptozotocin, baseline blood glucose of rats was determined. After 48 hours, the blood glucose was again measured to ensure that rats are diabetic. The induction of diabetes was confirmed if the blood glucose level doubled. Glucose was determined by a Glucometer (Infopia Co., Korea). Determination of blood glucose continued every 5 days to ensure the subsistence of diabetes. Regarding the entity of streptozotocin-induced diabetes, the animals which lost much weight and became weak, and those with uncertain blood glucose levels were excluded from the study. A total of 14 rats were used with equal numbers being assigned to control and test groups. Test group had 0.5 ml of solution comprising the herbal extract according to the present invention applied and the control group was dressed with distilled water. At time = 0 days, a full-thickness, circular 15 mm diameter wound was created (according to Wound Rep. Reg. 2002; 10: 286-294). Rats were anaesthetised by intraperitoneal pentobarbital (55 mg/kg) and the dorsal skin prepared for surgery using Betadine. The wound was created using surgical scissors. At time = 0 days dressings were placed, as prepared, directly on the wounds. The wounds were covered by sterile gases and wrapped carefully. Every 2-3 days following surgery, wounds were redressed with fresh control or test dressings while the rats were under anesthesia. The wounds were flushed with sterile saline to remove debris and to clean the wound area. A digital camera was used to take the pictures of the wound. The pictures were examined for wound healing in terms of wound size and appearance of new fresh epithelium. Once photographed, fresh dressings were placed on the wounds, and the wounds were covered again. Control of bias was achieved by assigning a code to each of the experimental groups. Investigators were blinded to the identity of each of the groups and the test and control had a similar appearance. The code was broken following completion of the final 4-week analysis. All animal experimentation was conducted under appropriate procedures approved by the UMDNJ IACUC review committee.

Figures 2 to 5 show photos which were taken during *in vivo* testing of the herbal extract according to the present invention on dermal wounds in the diabetic rat model. The herbal extract was applied in a form of dressings containing a solution thereof covering the wounds of the test group animals.

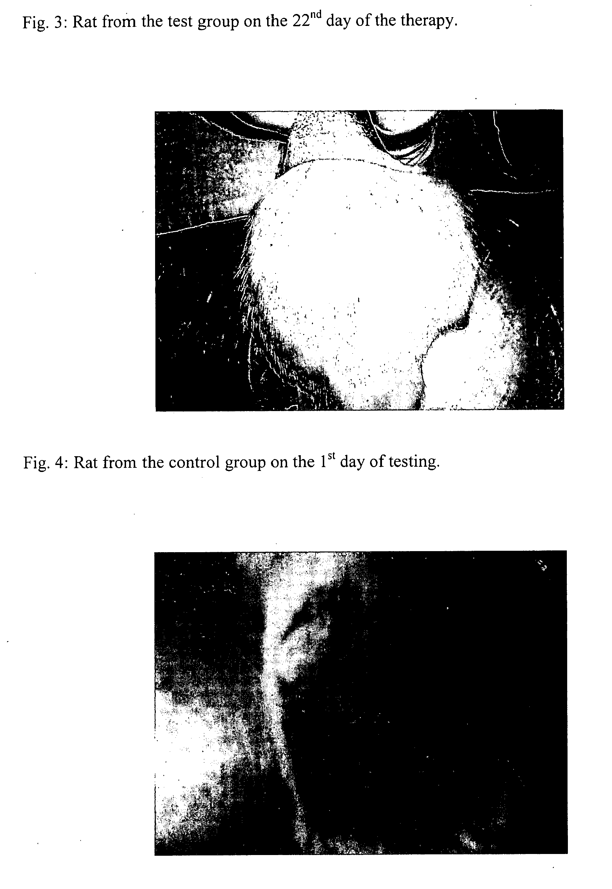

Figures 2 and 3 present pictures of rats from the test group (treated with the herbal extract) taken on the 1^{st}, and 22^{nd} day of therapy, respectively. In the test group on the 15^{th} day of therapy the wound is completely closed and the new, short fur covers the scar area. On the 22^{nd} day of therapy the wound is completely healed and the new, long fur covers the entire scar area. No signs of the previous wound could be seen.

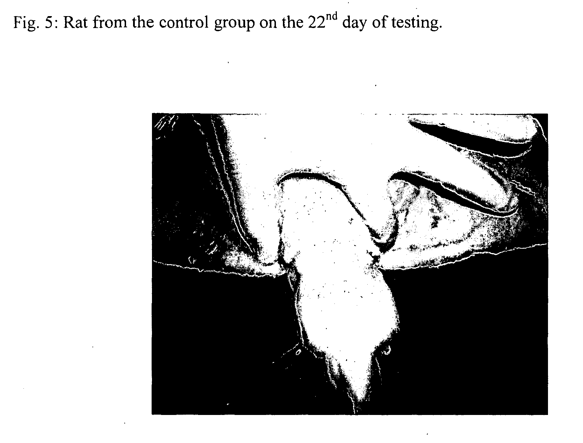

Figures 4 and 5 present pictures of rats from the control group (not treated) taken on the 1^{st}, and 22^{nd} day of therapy, respectively. In the control group on the 15^{th} day of therapy the wound is not closed. On the 22^{nd} day of testing the wound is closed but the scar is still remarkably severe and completely naked.

### Effects of the herbal extract on wound healing in vivo

As shown in the figures, wound areas and perimeters were similar in test and control groups; however, there was a tendency for more rapid closure in the test group, particularly at day 15 where the difference in wound areas and perimeters was most pronounced. The time to complete closure of wounds was lower in herbal extract-treated animals. In both control and test groups, wound area began to decrease at day 9^{th} and approximately complete wound closure first occurred by day 15^{th} (one out of seven rats). By day 22^{nd} wounds were essentially closed in both groups but growth of fur in the herbal extract-treated group was especially complete as compared to the water-treated group.

### Conclusion

The results of this study suggest that dermal preparation comprising the herbal extract according to the present invention has potential to enhance wound healing. However, rather than accelerating wound closure, herbal extract in this study appears to improve the quality of the tissue in the healing wound since the fur grew more efficiently than in the control group. Chronic wounds are not only characterized by untimely healing but also by the inability to remain closed following healing, thus time to closure may not be the only relevant end point or sole basis for efficacy of the treatment.

Obtaining the healthier scar tissue in the test group animals treated with the herbal extract allows anticipating a lowered recurrence rate.

### EXAMPLE 5: Assessment of the maximum tolerated dose (MTD) of the herbal extract in patients with diabetic foot ulcers (Phase I of Clinical Trials)

The main goal in treatment of the diabetic foot is to heal the ulcer. Many therapies are proposed and used for diabetic foot but all of them have a partial effect in ulcer improvement and prevention of amputation. More effective therapies are needed. Thus, the injectable herbal extract according to Example 2 has been introduced in treatment and progression in healing of diabetic foot. The main goal of this study was assessment of the maximal tolerated dose of this extract, injected intravenously to treat the diabetic foot ulcer.

A phase I of clinical trials was conducted with an open label, two stages design without control group. 6 Male patients over 18 years old with diabetes, without excluding criteria participated after signing the informed consent.

The patients were treated with intravenous administration of the herbal extract according to Example 2 diluted in 100 ml of sodium chloride 0.9% solution during one-hour. Primary dose was 2 ml daily for 28 days, which was escalated in next stages.

All 6 patients were evaluated for assessment of the DLTs (Dose Limiting Toxicities) of this extract.

Among 4 patients in escalated doses of extract, 2 ml, 4 ml, 6.7 ml and 10 ml daily for 28 days, respectively, no side effect was observed according to clinical and laboratory evaluations.

In the 5^{th} level of escalated dose of herbal extract, 13.5 ml daily, the patient showed phlebitis in injection place, on the 8^{th} day of treatment. After changing the injection place, the phlebitis was continued with the same dose and disappeared after decreasing the dose to 6.7 ml.

Phlebitis was observed again with 13.5 ml/day in the second patient of this level of dose escalation, so the extract was continued with 6.7 ml/day. No other side effect was observed with 13.5 ml/day dose.

**Table 1: Therapy groups and observed side effects**

| **Patient's Name** | **Daily dose** | **Restricting side effect (DLT)** |
|---|---|---|
| M.B. | 2 ml | None |
| Z.M. | 4 ml | None |
| M.H. | 6.7 ml | None |
| Gh.A. | 10 ml | None |
| M.H. | 13.5 ml | Phlebitis |
| D.N. | 13.5 ml | Phlebitis |

Six patients were treated with escalated doses which begun with 2 ml/day. The doses were easily tolerated up to 10 ml/day and no side effect or DLT was detected. Two cases showed phlebitis with 13.5 ml/day which was reviled with decreasing the extract up to 6.7 ml/day. Other clinical and laboratory evaluations demonstrated no side effects up to 10 ml/day. It is concluded that the dose of 10 ml/day was appropriate for Maximal Tolerated Dose (MTD) and DLT was only local phlebitis in the injection place.

The herbal extract of Example 2 is a safe drug with no side effects other than phlebitis and can be used with high confidence in patients with diabetic foot ulcers.

### EXAMPLE 6: Pharmacological effects of the herbal extract on diabetic foot ulcer (Phase II Clinical Trial)

### Patients and Methods

This study was conducted in Endocrine and Metabolism Research Center, Shiraz University of Medical Sciences, Shiraz, Iran. Patients were eligible for enrolment in the study if they met the following inclusion criteria: 18 years or older with a diabetic foot ulcer of at least 30 days' duration and an area of at least 1 cm² (greatest length × greatest width). The main exclusion criteria included the following: clinical signs of infection; a target wound that had exposed bone; a concurrent illness or a condition that may have interfered with wound healing (e.g., carcinoma, vasculitis, connective tissue disease, or an immune system disorder); known current abuse of alcohol or other drugs or treatment with dialysis, corticosteroids, immunosuppressive agents, radiation therapy, or chemotherapy, known hypersensitivity to any drugs; unwillingness or inability of an ambulatory patient to be fitted with appropriate shoe gear or an off-loading device.

After informed consent, at the baseline/initial visit, a full medical history and assessment of the patient's present conditions were obtained and recorded. Concomitant medications and their indications for use were also recorded. The diabetic status of the patient, including duration, type, and management, was noted with current activity level, ambulatory status, and history of ulceration or amputations.

Blood test results included levels of glycosylated haemoglobin, glucose, albumin, creatinine, serum urea nitrogen, and alkaline phosphatase; liver function; and human chorionic gonadotropin levels in women of reproductive age. The target wound's greatest length and width was measured at baseline. The target wound was photographed at baseline and then monthly. The target wound was assessed before and after cleansing and/or debridement for local infection and for wound condition (improving, stable, or deteriorating). Surgical debridement of healthy tissue was performed in the studied ulcer during the initial and all follow-up visits when necessary.

The wound area was determined by means of planimetry (the greatest length × the greatest width, measured in centimetres). The wounds were cleansed with isotonic sodium chloride solution at the time of the dressing change. The patient and/or health care provider were instructed on dressing change procedures. All patients were instructed to limit weight bearing, ambulating only for necessary activities.

### Protocol of drugs

The herbal extract of Example 2 was used as a daily i.m. injection (0.4 ml/day), slowly.

### Follow-up evaluations

Follow-up evaluations were completed on every two week basis. At each clinic visit, the investigator assessed and recorded the following: the study wound, compliance with drug use, the use of foot gear and/or off-loading, and the presence or absence of any adverse events.

### Results

The obtained results are presented on Fig. 1 as a chart of the mean ulcer area (cm²) of a patient at start, after one and two months of therapy. The differences between each pair are statistically significant (P value 0.0001, 0.002, and 0.009).

10 individuals (7 men and 3 women) who met our criteria enrolled to study. Mean surface area of ulcers were at start (12.32±11 cm²), after one month (9.55±9 cm²), and after two months (6.96±6 cm²), p<0.05.

We found no adverse effect in our patient. The laboratory data including; glucose, albumin, creatinine, serum urea nitrogen, alkaline phosphatase, and liver function showed non-significant changes after treating with above-mentioned drug.

### EXAMPLE 7: A randomized clinical trial:

### Evaluation of therapeutic effects of oral, topical and combination therapy of the herbal extract on patients with diabetic foot ulcer

Diabetic foot ulcer is one of the major causes of morbidity and mortality among diabetic patients.

It is prevalent among diabetics and is reported approximately 4.6% to 12%. Unfortunately, a large number of diabetic foot patients need amputation of foot or the lower limb.

Recent therapies have partial effects on wound healing and prevention of amputation. There is a high demand for more effective drugs. The goal of this clinical trial was to evaluate the therapeutic effects of the oral, topical and combination of the oral and topical forms of the herbal extract according to the present invention in diabetic foot, which can introduce a new way in treating this complication.

In the oral group, the patients were treated with the dry form of herbal extract packed in capsule form containing 100 mg active material each. Applied dose of the extract was 2 capsules daily (in two divided doses) for 45 days.

In the topical group, the patients were treated with the ointment form of herbal extract prepared as 3% concentration on the base of Vaseline. Applied dose of the extract was a thin layer of the ointment on the bed of ulcer and a thick layer around it, two times daily leaving the ulcer undressed at least 1 hour each time for 60 days.

In the combination group, the patients were treated with the oral and topical forms of the herbal extract as mentioned above simultaneously for 45 days.

All patients were followed during the therapy in two-weekly periods and also two months after the end of treatment course.

Patients were treated with standard methods as needed. Standard therapies and guidelines were applied in all study groups such as povidone iodine bath, antibiotic treatment when necessary; wound debridement, off-loading, correction of foot deformities, *etc.* All patients participated after signing the informed consent form.

Eighteen patients (6 in each treatment group) had been recruited in the trial and all of them terminated the therapy period successfully.

All patients had type 2 diabetes aged between 38 and 69 years old. Mean and standard deviation (SD) of wound size decrease at the end of treatment and two months after that comparing to the baseline were: 211±210 and 333±296 in oral group, 192±210 and 529±442 in topical group, and 500±404 and 779±477 in combination group, respectively. All of the above comparisons and wound size reductions in each group were statistically significant (p<0.05). There was no significant difference in wound healing and wound size reduction between three treatment groups (p>0.15).

As a conclusion, the results of these 18 patients demonstrated the complete effect of all types of oral, topical and combination of these two forms of drug in healing of diabetic foot ulcer and granulation tissue formation.

The ulcers in most of patients were completely closed and its diameter in other patients was decreased significantly. In deed, all of patients, except one, had proper to excellent response to the treatment and mean size of the ulcers was decreased more than 80% up to two months after treatment. In the period of the therapy no side effects were observed in clinical examinations, laboratory analysis and other evaluations.

Some results are shown in Figures 6 to 11.

According to the results of this trial, it could be concluded that the herbal extract according to the present invention has many advantages to available therapies as follows:
- It is completely safe in usual doses (no side effects was observed with usual, effective dose).
- Completely effective in diabetic foot ulcer (complete improvement in most of the patients).
- Applicable in all patients with diabetic foot (all patients respond to this extract).
- The therapeutic effects appear in a short period of time (in 2 weeks).
- With decreasing the hospitalisation and fast improvement of the ulcer, direct and indirect costs are decreased dramatically.

### EXAMPLE 8: A multi center randomized clinical trial:

### Evaluation of therapeutic effects of the herbal extract

### on patients with diabetic foot ulcer (Phase III of Clinical Trials)

Long term complications of diabetes are one of the major causes of morbidity and mortality among diabetic patients.

The prevalence of diabetic foot in diabetics is reported approximately 4.6% to 12%. Unfortunately, a large number of diabetic foot patients need amputation of foot or the lower limb.

Recent therapies have partial effects on wound healing and prevention of amputation. There is a high demand for more effective drugs. The goal of this clinical trial was to evaluate the therapeutic effects of the injectable form of the herbal extract according to the present invention in diabetic foot, which can introduce a new way in treating this complication.

The patients were treated with intravenous injection of the herbal extract according to Example 2 diluted in sodium chloride 0.9 % solution. Applied dose of the extract was 4 ml daily in 100 ml normal saline infused in ½ hours for 28 days. All patients were followed during the therapy period.

In the control group, the patients were treated with standard methods. Standard therapies and guidelines were applied in both study groups such as povidone iodine bath, antibiotic treatment when necessary; wound debridement, off-loading, correction of foot deformities, *etc.* All patients were participated after signing the informed consent form.

Twenty six patients (15 in intervention group and 11 in control group) had terminated the therapy period and the present results are an interim analysis. Primary results of these 26 patients demonstrated the complete effect of the drug in improvement of diabetic foot ulcer, granulation tissue formation, improvement in microvascular circulation and preventing from amputation in 15 patients with the new herbal extract treatment

The ulcers in most of patients were completely closed and its diameter in other patients was decreased significantly. One month after beginning the standard treatment, the mean size of the ulcers was decreased less than 12% in controls (p<0.05). In the period of the therapy no side effects were observed in clinical examinations, laboratory analysis and other evaluations.

Some results are shown in Figures 12 to 13.

According to the results of the primary analyses, it seems that the herbal extract according to the present invention has many advantages to available therapies as follows:
- It is completely safe in usual doses (no side effects was observed with usual, effective dose).
- Completely effective in diabetic foot ulcer (complete improvement in most of the patients).
- Applicable in all patients with diabetic foot (all patients respond to this extract).
- The therapeutic effects appear in a short period of time (in 2 weeks).
- With decreasing the hospitalisation and fast improvement of the ulcer, direct and indirect costs are decreased dramatically (the mean hospitalisation time for diabetic foot ulcer in Iran is approximately 3.8 weeks, but in this study the patients were hospitalised for approximately 8 days).
- In many occasions, the extract can prevent amputation (2 cases underwent the therapy were candidates for amputation which were treated and after the therapy, the amputation was not necessary. In the control group, one patient had the same situation and his limb was amputated after 5 days).

### EXAMPLE 9: A randomized clinical trial:

### Determining the effect of the intravenous herbal extract on patients with bed sores

The main goal is to determine the effects of the intravenous herbal extract according to Example 2 in treatment of patients with bed sores and compare it with the standard treatment.

Patients include adult male and female subjects (with age more than 18 years) with bed sores. All patients with ulcers resulted from spinal complications (accidents or congenital), amputation of the lower limbs, chronic diseases like brain vessel disorders or fractures due to osteoporosis are included. Their ulcer, sizes must be at least 1 cm² (the longest length the longest width) and it must be occurred at least 2 weeks ago.

18 patients, 9 in intervention and 9 in control group completed the trial. The mean age of the intervention group was 44.7 ±19 years and the controls 46.7±22.7 years, Male to female ratio was 7 to 2 in each group. The ulcer area at the beginning, in the intervention group was 35.1 ±30.1 cm² which changed to 12.2 ±19 after treatment (P=0.004). The ulcer area in the control group was 17.6 ±17.3 at the beginning, and 14.8 ±14.5 at the end of therapy period (P=0.205).

**Table 2: Comparison of bed sore healing between intervention and control group**

| **Healing percentage** | **Intervention group %(n)** | **Control group %(n)** | **P value** |
|---|---|---|---|
| **Complete healing** | 55.6% (5) | 0%(0) | 0.011 |
| **(100%)** | | | |
| **Proper healing** | 11.1%(1) | 11.1%(1) | |
| **(50-75%)** | | | |
| **Partial healing** | 33.3% (3) | 22.2%(2) | |
| **(<50%)** | | | |
| **No healing (0%)** | 0%(0) | 66.7%(6) | |

Unfortunately, there is no complete and perfect treatment for bed sores without surgery or bed sores after surgical debridement. It is clear that we need a drug which can induce wound healing with minimum side effects. According to the results of the present study on 18 patients, the healing percentage of the ulcer in the treated group was significantly more than other patients in control group. The results of this trial demonstrated that the herbal extract of the present invention can account as a new and effective drug in treatment of bed sores and can improve the patients' quality of life significantly. Thus the need for extensive debridement and graft implication are reduced. Finally, it is concluded that the herbal extract of the present invention can improve the treatment of bed sores significantly, thus can be used as a new and effective drug in bed sores and pressure ulcers.

The features disclosed in the foregoing description, in the claims and in the drawings may, both separately and in any combination thereof, be material for realizing the invention in diverse forms thereof.

## Claims

1. A method for preparing a herbal extract, comprising the following steps:
(a) providing a plant material derived from *Mellilotus sp,*;
(b) drying the plant material;
(c) adding an organic solvent;
(d) incubating the mixture of plant material and organic solvent; and
(e) obtaining the herbal extract.

2. The method according to claim 1, wherein the plant material is derived from *Mellilotus officinalis.*

3. The method according to claim 1 or 2, wherein the plant material derived from *Mellilotus officinalis* comprises leaves and/or small stems.

4. The method according to any of the preceding claims, wherein the drying in step (b) is carried out at a temperature in the range of about 20 to 50°C, preferably at about 37 to 45°C, most preferably at about 42°C.

5. The method according to any of the preceding claims, wherein the drying in step (b) is carried out for a time period of about 3 to 4 days.

6. The method according to any of the preceding claims, wherein the organic solvent is ethanol, preferably of about 60 to 96 % (by volume), more preferably of about 80 to 96 % (by volume), most preferably of about 96 % (by volume).

7. The method according to any of the preceding claims, wherein the incubating in step (d) is carried out for a time period in the range of about 10 to 40 days, preferably of about 22 to 38 days, most preferably of about 25 to 35 days.

8. The method according to any of the preceding claims, wherein the incubating in step (d) is carried out at a temperature in the range of about 20 to 50°C, preferably of about 37 to 45°C, most preferably of about 42°C.

9. The method according to any of the preceding claims, wherein the method additionally comprises the following step:
(f) adding urea.

10. The method according to any of the preceding claims, wherein the method additionally comprises the following step:
(g) adding fructose.

11. The method according to any of the preceding claims, wherein the method additionally comprises the following step:
(h) adding phosphoglycerol and/or its sodium salt.

12. The method according to any of the preceding claims, wherein the method additionally comprises the following step:
(i) adding selenium and/or an organic or inorganic salt thereof.

13. The method according to claim 12, wherein the selenium or salt thereof is added to a concentration of free selenium in the range of about 1 to 100 mg/l, preferably of about 5 to 50 mg/l, most preferably of about 10 to 20 mg/l.

14. The method according to any of the preceding claims, wherein the method additionally comprises the following step:
(j) exposing the herbal extract to a pulsed electromagnetic field.

15. The method according to claim 14, wherein the electromagnetic field pulse has a sinusoidal, rectangular and/or stochastic shape.

16. The method according to claim 14 or 15, wherein the pulsed electromagnetic field has a frequency in the range of about 5 to 750 kHz, preferably of about 50 to 350 MHz, most preferably of about 250 MHz.

17. The method according to any of claims 14 to 16, wherein the pulsed electromagnetic field has a power in the range of about 10 to 200 Watt, preferably of about 20 to 100 Watt, most preferably of about 45 Watt.

18. The method according to any of claims 14 to 17, wherein the pulsed electromagnetic field has a magnetic field strength in the range of 100 to 150 µTesla.

19. The method according to any of claims 14 to 18, wherein the exposing in step (j) is carried out for a time period of about 3 to 5 minutes.

20. The method according to any of claims 14 to 19, wherein the exposing in step (j) is repeated, and is preferably carried out for three times.

21. A herbal extract obtainable by the method according to any of claims 1 to 20.

22. A use of the herbal extract according to claim 21 or prepared by the method according to claims 1 to 20, for the treatment of chronic wounds in a subject.

23. A use of the herbal extract according to claim 21 or prepared by the method according to claims 1 to 20, for the manufacture of a pharmaceutical composition for the treatment of chronic wounds in a subject.

24. The use according to claims 22 or 23, wherein the chronic wounds are associated with states in which the normal wound repair ability is weakened.

25. The use according to any of claims 22 to 24, wherein the chronic wounds are associated with states such as diabetes and/or occur in patients in their older age or during steroid treatment.

26. The use according to any of claims 22 to 25, wherein the chronic wounds are diabetic foot ulcers, neuropathic ulcers including neuropathic forefoot ulcers, diabetic pressure ulcers or diabetic venous ulcers; bed sores or pressure ulcers associated with long-term disability.

27. The use according to any of claims 22 to 26, wherein the chronic wounds are diabetic foot ulcers.

28. The use according to any of claims 22 to 26, wherein the chronic wounds are bed sores.

29. The use according to any of claims 22 to 28, wherein the subject is a vertebrate, preferably a mammal, most preferably a human.

30. The use according to any of claims 22 to 29, wherein the subject is not pregnant.

31. A pharmaceutical composition comprising the herbal extract according to claim 21 or prepared by the method according to claims 1 to 20.

32. The pharmaceutical composition according to claim 31, additionally comprising a pharmaceutically acceptable carrier.

33. The pharmaceutical composition according to claims 31 or 32, formulated for administration by injection.

34. The pharmaceutical composition according to claims 31 or 32, formulated for oral administration.

35. The pharmaceutical composition according to claims 31 or 32, formulated for topical administration.

36. A kit comprising the pharmaceutical composition according to any of claims 31 to 35.
